# EUROPEAN PATENT SPECIFICATION

(11) **EP 4 284 235 B1**
(45) Date of publication and mention of the grant of the patent: **04.11.2026**
(21) Application number: 22746558.0
(22) Date of filing: 27.01.2022
(51) Int. Cl.: A61B 5/055, G16H 30/40, G16H 40/67, G16H 50/20, G01R 33/56, G16H 50/30

(54) **IMAGE-BASED APPROACH TO EVALUATE CONNECTIVE TISSUE STRUCTURE, REMODELING, AND RISK OF INJURY**
BILDBASIERTER ANSATZ ZUR BEURTEILUNG DER BINDEGEWEBESTRUKTUR, NEUMODELLIERUNG UND VERLETZUNGSRISIKO
APPROCHE BASÉE SUR UNE IMAGE POUR ÉVALUER LA STRUCTURE DU TISSU CONJONCTIF, LE REMODELAGE ET LE RISQUE DE LÉSION

(30) Priority: 28.01.2021 US 202163142974 P
(43) Date of publication of application: 06.12.2023
(73) Proprietor: Children's Medical Center Corporation, Boston, MA 02115 (US); Rhode Island Hospital, Providence, RI 02903 (US)
(72) Inventor: KIAPOUR, Ata, Wayland, MA 01778 (US); FLEMING, Braden, C., Sherborn, MA 01770 (US); MURRAY, Martha, M., Sherborn, MA 01770 (US)
(74) Representative: Vossius & Partner Patentanwälte Rechtsanwälte mbB
(86) International application number: PCT/US2022/013979
(87) International publication number: WO 2022/164952

(56) References cited:
- WO-A1-2018/204404
- KR-A- 20140 141 055
- US-A1- 2013 084 246
- US-A1- 2020 069 257
- MONU U D ET AL: "Cluster analysis of quantitative MRI T2 and T1[rho] relaxation times of cartilage identifies differences between healthy and ACL-injured individuals at 3T", OSTEOARTHRITIS AND CARTILAGE, ELSEVIER, AMSTERDAM, NL, vol. 25, no. 4, 5 October 2016 (2016-10-05), pages 513 - 520, XP029957374, ISSN: 1063-4584, DOI: 10.1016/J.JOCA.2016.09.015
- NIKAN K. NAMIRI; IO FLAMENT; BRUNO ASTUTO; RUTWIK SHAH; RADHIKA TIBREWALA; FRANCESCO CALIVA; THOMAS M. LINK; VALENTINA PEDOIA; SHA: "Hierarchical Severity Staging of Anterior Cruciate Ligament Injuries using Deep Learning with MRI Images", ARXIV.ORG, CORNELL UNIVERSITY LIBRARY, 201 OLIN LIBRARY CORNELL UNIVERSITY ITHACA, NY 14853, 20 March 2020 (2020-03-20), 201 Olin Library Cornell University Ithaca, NY 14853 , XP081644065

## Description

### FIELD OF THE DISCLOSURE

The present disclosure relates to techniques for magnetic resonance imaging biological tissues.

### BACKGROUND

Magnetic resonance imaging (MRI) is a non-invasive and versatile technique for imaging biological systems. Generally, MRI operates by detecting magnetic resonance (MR) signals, which are electromagnetic waves emitted by atoms in response to an applied electromagnetic field. The detected MR signals may then be used to generate MR images of tissues of a patient, usually internal to the patient and unable to be directly viewed without invasive surgery.

Connective tissue condition determination based on magnetic resonance images, according to the state of the art, is for instance also described in WO2018/204404A1, KR20140141055A, as well as: MONU ET AL: "Cluster analysis of quantitative MRI T2 and T1ρ relaxation times of cartilage identifies differences between healthy and ACL-injured individuals at 3T", OSTEOARTHRITIS AND CARTILAGE, ELSEVIER, AMSTERDAM, NL, vol. 25, no. 4, 5 October 2016, pages 513-520, ISSN: 1063-4584, DOI: 10.1016/J.JOCA.2016.09.015.

### SUMMARY OF THE INVENTION

According to the invention, there are provided a method of determining a condition of a connective tissue of a patient from analysis of a magnetic resonance image according to claim 1 and a corresponding computer system according to claim 15, preferred embodiments being specified in dependent claims 2-14.

### SUMMARY OF THE DISCLOSURE

In one embodiment, there is provided a method of determining a condition of a tissue of a patient from analysis of a magnetic resonance image, the method comprising: generating a projection from a magnetic resonance image depicting the tissue, wherein generating the projection comprises determining a value at a point in the projection based on at least one value at a position of the magnetic resonance image corresponding to the point in the projection; determining, for each of a plurality of locations in the projection, the condition of the tissue at a location, wherein determining the condition of the tissue at the location comprises determining the condition based at least in part on at least one value of the projection at the location; and outputting the determined condition at the plurality of locations of the projection.

In another embodiment, there is provided a computer system, comprising: at least one processor; and a non-transitory computer-readable storage medium storing executable instructions that, when executed by the at least one processor, cause the at least one processor to perform the method of determining a condition of a tissue of a patient from analysis of a magnetic resonance image.

In another embodiment, there is provided at least one non-transitory computer-readable storage medium storing executable instruction that, when executed by at least one processor, cause the at least one processor to perform the method of determining a condition of a tissue of a patient from analysis of a magnetic resonance image.

The foregoing summary is to be considered non-limiting.

### BRIEF DESCRIPTION OF DRAWINGS

The accompanying drawings are not intended to be drawn to scale. In the drawings, each identical or nearly identical component that is illustrated in various figures is represented by a like numeral. For purposes of clarity, not every component may be labeled in every drawing. In the drawings:
FIG. 1 is a schematic diagram of a magnetic resonance imaging (MRI) system for performing an image-based approach to guide postoperative care of patients with connective tissue injury, in accordance with some embodiments described herein;
FIG. 2 is a flowchart of an illustrative process 200 of determining a condition of tissue of a patient from analysis of a magnetic resonance image, in accordance with some embodiments described herein;
FIG. 3 is a flowchart of an illustrative process 300 of generating a projection from a magnetic resonance image, in accordance with some embodiments described herein;
FIG. 4 is a flowchart of an illustrative process 400 of determining a condition of tissue at locations in a projection of the magnetic resonance image, in accordance with some embodiments described herein;
FIG. 5A is an illustrative example of a process of generating a healing map from a magnetic resonance image, in accordance with some embodiments described herein.
FIG. 5B is an illustrative example of healing maps generated at times after an anterior cruciate ligament reconstruction, and healing maps generated at times after a bridge-enhanced anterior cruciate ligament repair, in accordance with some embodiments described herein;
FIG. 5C is an illustrative example of the progression of the postoperative changes in the volume of the tissue with low quality in the months following an anterior cruciate ligament reconstruction and a bridge-enhanced anterior cruciate ligament repair, in accordance with some embodiments described herein;
FIG. 5D is an illustrative example of surgically treated anterior cruciate ligaments that experienced subsequent reinjury, in accordance with some embodiments described herein; and
FIG. 6. is a schematic diagram of an illustrative computing device with which aspects described herein may be implemented, in accordance with some embodiments described herein.

### DETAILED DESCRIPTION

Described herein are techniques to aid clinicians and researchers in determining a condition of connective tissue as it relates to tissue development, growth and maturation, tissue remodeling and healing following injury, and risk of injury based on a magnetic resonance (MR) image of the tissue. Such techniques may be useful to clinicians by providing insights on factors that influence the growth and maturation of connective tissues as well as those that impact the risk of connective tissue injury and response to treatment. These insights can be used in a variety of ways, including to guide or develop patient specific risk assessment and prevention strategies, treatment plans, and postoperative care plans for individuals at risk of connective tissue injuries and those with injured connective tissues, such as an anterior cruciate ligament (ACL) injury.

Some techniques described herein utilize a magnetic resonance image-based approach to evaluate tissue structure, which may include methods for quantifying connective tissue healing and determining location specific conditions of a tissue that may correspond to a quantitative score and/or a 2D map indicative of an overall condition of the tissue. This may include assessing location specific conditions of a tissue from an MR image. More particularly, in some embodiments, the methods include determining the condition of tissue and the spatial variation of the condition across the tissue for determining the status of tissue health, strength, remodeling, risk of injury and/or recovery after injury and treatment for a patient. As discussed in more detail below, some techniques described herein include generating a projection from an MR image of an ACL or other connective tissue. The projection may then be analyzed to determine the condition of tissue, rather than needing to analyze the complex MR images directly. Advantageous techniques are described herein for generation and analysis of such a projection. As also discussed in more detail below, locations of the projection may be annotated to indicate the status of tissue health, strength, remodeling, and risk of injury at the corresponding location of tissue. Accordingly, the annotated projection provides a simplified representation of the condition of the tissue to enable a clinician to quickly and accurately analyze the projection to make a determination on the condition of the tissue. Additionally, the annotations may emphasize areas relevant to a condition of the tissue using color-coding to indicate the spatial variation of the condition of the tissue as a qualitative and a quantitative metric a clinician may use for determining status of tissue health, strength, remodeling, risk of injury, and/or recovery after injury and treatment for a patient.

Traumatic injuries of joint connective tissue are among the most common musculoskeletal injuries in adolescents and adults participating in physically demanding activities such as sports and military operations. There are more than 17 million joint sprain and strain injuries treated in the U.S. annually. These injuries are often immediately disabling, expensive to treat, and may lead to lasting complications such as joint degeneration. For example, rupturing of the ACL is one of the most common and devastating connective tissue injuries, primarily occurring to young, active individuals. These injuries result in more than 400,000 ACL surgeries each year in the U.S. alone.

In many cases, the rate of ACL reinjuries following surgery can be up to 40%, due in part to a lack of effective clinical assessments that have the appropriate sensitivity and specificity to determine when a patient is at a high risk of reinjury. Accordingly, following an ACL surgery, a major challenge in designing a patient's care plan is determining what types of activities are safe to engage in during various periods of the healing process without risking a reinjury of the ACL. Contemporary clinical assessments for evaluating the ACL during the healing process include clinical examinations (e.g., range of motion), functional tests (e.g., balance), and patient reported outcomes. These assessments may be influenced by factors unrelated to the healing ACL and are prone to observer bias. As such, despite these assessments being widely used, they often fail to reduce the risk of ACL reinjury, due to the low sensitivity and lack of specificity associated with the clinical assessments. The high reinjury rate is due, in part, to the difficulty of assessing the extent to which an ACL has healed following a reconstructive surgery. Without having a precise determination of the health of the tissue, it is challenging to determine the risk of reinjury associated with patient activity. For example, development of an effective patient-specific post-operative care plan, including approving a return to sport, following an ACL surgery is one of the most complex tasks a sports medicine team may face.

Direct assessment of ACL tissue quality could mitigate these shortcomings by providing high sensitivity and patient specific results. The traditional methods of direct assessment used in preclinical studies include comparing biomechanical and histological outcomes. However, due to the destructive nature of these techniques, they are not suitable for clinical studies. A noninvasive, nondestructive technique that allows direct assessment of the ACL could significantly improve post-operative patient outcomes.

MRI is one such noninvasive, nondestructive technique that if used to evaluate the condition of tissue of the ACL could provide advantages over the existing clinical assessments. MR imaging may be used to generate 2D or 3D images of a patient's knee, providing a clinician with a representation of the morphological structure of the tissue. In cases of severe damage, a rupture may be diagnosed from the morphological features of the tissue. Swelling, changes in the shape, or even changes in the orientation of the ACL may be indicative of damage.

Some health conditions of the tissue may appear as different intensities and quantitative metrics (i.e. phase, spatial frequencies, and/or relaxation times) in an MR image. In the case of severely damaged tissue, the intensity discrepancies between the healthy tissue and unhealthy tissue may be obvious and thus may be used to diagnose an injury. However, despite MRI sometimes being used as a technique for diagnosing a tear in an ACL, it is not straightforward to identify a condition, such as the strength, tissue remodeling, healing, or response to treatment, of recovering tissue from an MR image. This is due, in part, to the quantitative differences, in a recovering ACL, between healthy tissue and unhealthy tissue being potentially less pronounced and thus more difficult to identify and morphological features potentially having no distinguishing characteristics between heathy and unhealthy tissue. Further, the complex differences and variation in morphological and structural features of a healing tissue are extremely challenging to observe or detect in MRI. As such, the complex analysis required to analyze MR images requires specialized training, may further be time intensive, and even so may be unreliable.

As such, MR imaging has not conventionally been used to predict long term clinical and functional outcomes following ACL surgery. Several studies have tried to use MRI to track changes in ACL health, however, they have not been successful. Such earlier approaches to use MRI to predict long term clinical and functional outcomes have relied on a global measure of the ACL quality, by looking at the whole tissue. As a result, such techniques are insensitive to the distribution of healthy and unhealthy tissue within the ACL and therefore suffer from low accuracy and have been unsuitable for determining long term patient outcomes. More specifically, conventional methods that use MR images to determine risk of reinjury evaluate the average intensity (or another global measure of the tissue) corresponding to ACL tissue, and use that average intensity to determine the health of the ACL tissue. Because this analysis of "average" intensity (or other global measure) fails to account for the distribution of healthy and unhealthy tissue, it can overlook localized weaknesses in the tissue that can lead to reinjuries. For example, a first patient ACL may have several regions of inferior tissue quality distributed around regions of superior tissue quality, while a second patient ACL may have one large region of inferior tissue quality while the rest of the ACL may be superior tissue quality. These two images may reflect the same average intensity but may have drastically different responses to treatment and dramatically different risks of reinjury.

Despite the high prevalence of connective tissue injuries, and despite the substantial flaws and low accuracy of conventional techniques, both without MRI and with MRI, for determining risk of reinjury, those conventional techniques continue to be used.

The inventors have recognized and appreciated that analysis of signal distribution and patterns of signal distribution within a tissue may enable more accurate determination of status of tissue health, strength, remodeling, risk of injury, and/or recovery after injury and during or following treatment of a patient.

More particularly, the inventors have recognized and appreciated that such challenges may be mitigated by determining a condition of a tissue at multiple locations of the tissue in the MR image and examining the distribution of intensities across the tissue depicted in the MR image. Some distributions of the determined condition may result in an inferior response to treatment and ultimately lead to higher risk of reinjury than others. In particular, the condition of the tissue may be determined by the intensity of the ACL tissue and may be evaluated at multiple locations by comparing to a threshold intensity corresponding to a healthy condition of a tissue.

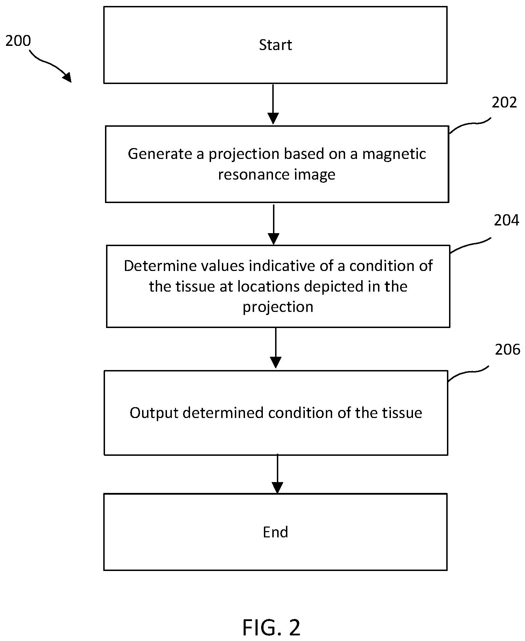

The inventors have accordingly developed systems and methods, described herein, to determine a condition of a tissue which can be used to assess tissue health status, healing response to treatment, and risk of injury based on a MR image of the tissue. In some embodiments, determining a risk of injury includes a mapping approach to track tissue healing and remodeling by determining a condition of a tissue at locations across a healing map. The healing map may be a visual representation of the tissue, with locations in the map annotated according to their signal relative to a threshold. For example, the healing map may document the complex 3D distribution of the locations of a condition across a tissue into a single 2D projection which can be used to assess tissue health status, healing response to treatment, and risk of injury. The inventors have recognized that generating a healing map from an MR image of the tissue may provide a clearer and more informative qualitative tool as well as a quantitative tool for evaluating the condition of the tissue. According to some embodiments, the condition of the tissue that is evaluated may include tissue remodeling, tissue strength, and/or risk of injury and the healing map may annotate the condition of tissue by color coding pixels corresponding to locations on the tissue based on their relative signal, as determined from the MR image, which can be used to assess location specific conditions of the tissue. A clinician can easily evaluate the tissue health and distribution of low-quality tissue from the healing map, thus the clinician has a qualitative and quantitative tool to aid in developing a proper risk assessment and patient care plan. Techniques for generating such healing maps are described herein including with reference to FIGs. 2-5.

Accordingly, some embodiments provide for a method of determining a condition of a tissue of a patient from analysis of a magnetic resonance image, with the method including generating a projection from a magnetic resonance image depicting a tissue. In some embodiments, generating the projection includes determining a value at a point in the projection based on at least one value at a position of the MR image corresponding to the point in the projection. A projection may be, for example, a two-dimensional (2D) representation of the tissue depicted in the magnetic resonance image, where the value at a point in the projection is based on the intensity of at least one position in the MR image.

In some embodiments, the method may include determining a condition of a tissue at a location for each of a plurality of locations in the projection. The determination may be based, at least in part, on at least one value of the projection at the location. As one example, the number of locations may correspond to the number of pixels or voxels in the magnetic resonance image, and the value at each location is compared to at least one threshold value indicative of the condition of the tissue.

In some embodiments, determining a condition of a tissue may comprise outputting the determined condition of tissue at the plurality of locations of the projection. For example, outputting the determined condition may include displaying a color-coded overlay on locations of the projections, where the colors correspond to different conditions of tissue for the patient.

The inventors have further developed an image analysis facility for determining a condition of a tissue from MRI data. In some embodiments, such MRI data may have been captured using an MRI system for acquiring the MR images, where the system includes a magnetics system configured to produce one or more magnetic fields during MR imaging and at least one radio frequency coil configured to produce one or more radio frequency pulses during MR imaging. One or more processors may be configured to receive a MR image of a patient who has received a surgery. The at least one processor may be further configured to determine the condition of the reconstructed tissue. In some cases, the MR image may be an MR image of a knee of a patient who has received ACL surgery, and the determination may be of the condition of the reconstructed ACL tissue.

Various examples of ways in which these techniques and systems may be implemented are described below. It should be appreciated, however, that embodiments are not limited to operating in accordance with these examples. Other embodiments are possible.

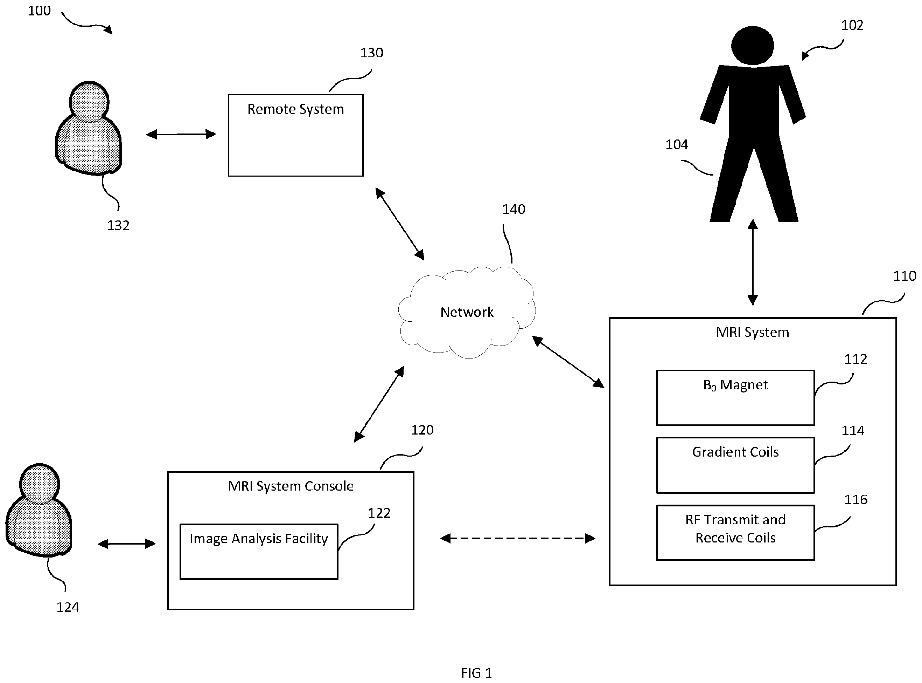

FIG. 1 is a block diagram of an example of a system 100 for determining a condition of a tissue, in accordance with some embodiments described herein. In the illustrative example of FIG. 1, system 100 includes an MRI system 110, and MRI system console 120, and a remote system 130. It should be appreciated that system 100 is illustrative and that a system may have one or more other components of any suitable type in addition to or instead of the components illustrated in FIG. 1. For example, there may be additional remote systems (e.g. two or more) present within a system.

As illustrated in FIG. 1, in some embodiments, one or more of the MRI system 110, the MRI system console 120, and the remote system 130 may be communicatively connected by a network 140. The network 140 may be or include one or more local and/or wide-area, wired and/or wireless networks, including a local-area or wide-area enterprise network and/or the Internet. Accordingly, the network 140 may be, for example, a hard-wired network (e.g., a local area network within a healthcare facility), a wireless network (e.g., connected over Wi-Fi and/or cellular networks), a cloud-based computing network, or any combination thereof. For example, in some embodiments, the MRI system 110 and the MRI system console 120 may be located within the same healthcare facility and connected directly to each other or connected to each other via the network 140, while the remote system 130 may be located in a remote healthcare facility and connected to the MRI system 110 and/or the MRI system console 120 through the network 140.

In some embodiments, the MRI system 110 may be configured to perform MR imaging of anatomy of a patient 102, such as a knee 104 of the patient in some scenarios. For example, the MRI system 110 may include a B0 magnet 112, gradient coils 114, and radio frequency (RF) transmit and receive coils 116 configured to act in concert to perform said MR imaging.

In some embodiments, B0 magnet 112 may be configured to generate the main static magnetic field, B0, during MR imaging. The B0 magnet 112 may be any suitable type of magnet that can generate a static magnetic field for MR imaging. For example, the B0 magnet 112 may include a superconducting magnet, an electromagnet, and/or a permanent magnet. In some embodiments, the B0 magnet 112 may be configured to generate a static magnetic field having a particular field strength.

In some embodiments, gradient coils 114 may be arranged to provide one or more gradient magnetic fields. For example, gradient coils 114 may be arranged to provide gradient magnetic fields along three substantially orthogonal directions (e.g., x, y, and z). The gradient magnetic fields may be configured to, for example, provide spatial encoding of MR signals during MR imaging. Gradient coils 114 may comprise any suitable electromagnetic coils, including discrete wire windings coils and/or laminate panel coils.

In some embodiments, RF transmit and receive coils 116 may be configured to generate RF pulses to induce an oscillating magnetic field, B1, and/or to receive MR signals from nuclear spins within a target region of the imaged subject (e.g. of the knee 104) during MR imaging. The RF transmit coils may be configured to generate any suitable types of RF pulses useful for performing MR imaging. RF transmit and receive coils 116 may comprise any suitable RF coils, including volume coils and/or surface coils.

As illustrated in FIG. 1, system 100 includes MRI system console 120 communicatively coupled to the MRI system 110. MRI system console 120 may be any suitable electronic device configured to send instruction and/or information to MRI system 120, to receive information from MRI system 120, and/or to process obtained MR data. In some embodiments, MRI system console 120 may be a fixed electronic device such as a desktop computer, a rack-mounted computer, or any other suitable fixed electronic device. Alternatively, MRI system console 120 may be a portable device such as a laptop computer, a smart phone, a tablet computer, or any other portable device that may be configured to send instructions and/or information to MRI system 120, to receive information from MRI system 120, and/or to process obtained MR data.

Some embodiments may include an image analysis facility 122. Image analysis facility 122 may be configured to analyze MR data obtained by MRI system 110 from an MR imaging procedure of patient 102. Image analysis facility 122 may be configured to, for example, analyze the obtained MR data by determining the condition of a tissue for one or more sets of MR data, as described herein. Image analysis facility 122 may be implemented as hardware, software, or any suitable combination of hardware and software, as aspects of the disclosure provided herein are not limited in this respect. As illustrated in FIG. 1, the image analysis facility may be implemented in the MRI system console 120, such as by being implemented in software (e.g., executable instructions) executed by one or more processors of the MRI system console 120. However, in other embodiments, the image analysis facility 122 may be additionally or alternatively implemented at one or more other elements of the system 100 of FIG. 1. For example, the image analysis facility 122 may be implemented at or with another device, such as a device located remote from the system 100 and receiving data via the network 140.

MRI system console 120 may be accessed by MRI user 124 in order to control MRI system 120 and/or to process MR data obtained by MRI system 120. For example, MRI user 124 may implement an MRI imaging process by inputting one or more instructions into MRI system console 120 (e.g., MRI user 124 may select an MR imaging process from among several options presented by MRI system console 120). Alternatively, or additionally, in some embodiments, MRI user 124 may implement an MR data analysis procedure by inputting one or more instructions into MRI system console 120 (e.g. MRI user may select MR data instances to be analyzed by MRI system console 120).

As illustrated in FIG. 1, MRI system console 120 also interacts with remote system 130 through network 140, in some embodiments. Remote system 130 may be any suitable electronic device configured to receive information (e.g., from MRI system 110 and/or MRI system console 120) and to display generated MR images for viewing. The remote system 130 may be remote from the MRI system 110 and MRI system console 120, such as by being located in a different room, wing, or building of a facility (e.g., a healthcare facility) than the MRI system 110, or being geographically remote from the system 110 and console 120, such as being located in another part of a city, another city, another state or country, etc. In some embodiments, remote systems 130 may be a fixed electronic device such as a desktop computer, a rack-mounted computer, or any other suitable fixed electronic device. Alternatively, remote system 130 may be a portable device such as a laptop computer, a smart phone, a table computer, or any other portable device that may be configured to receive and view generated MR images and/or to send instructions and/or information to MRI system console 120.

In some embodiments, remote system 130 may receive information (e.g., MR data analysis results, generated MR images of a patient, and/or raw MR data) from MRI system console 120 and/or MRI system 110 over the network 140. A remote user 132 (e.g., the patient's medical clinician) may use remote system 130 to view the received information on remote system 130. For example, the remote user 132 may view generated MR images using remote system 130 after the MRI user 124 has completed MR data analysis using MRI system 110 and/or MRI system console 120.

The inventors have recognized and appreciated that determining the condition of a tissue and generating a projection to convey the spatial distribution of the condition to a user could enable accurate and efficient determination of status of tissue health, the strength of ligament tissue, remodeling of the tissue, risk of injury, and/or recovery after injury and treatment for a patient. For example, when examining a 3D volumetric image, values associated with the surface of the tissue may obscure values associated with an interior of the tissue. Similarly, a 3D MR image can be examined as a 2D image corresponding to a slice through the tissue. Although examining a 2D image or a series of 2D images allows a clinician to examine MR data from the interior of a tissue, it is difficult to analyze features that are not located in the observed plane. It is also extremely challenging for a clinician to visualize and comprehend the spatial distribution of each voxel in a 3D manner. Thus, it is time consuming and can require specialized training to interpret a 3D image of a tissue. As such, the inventors have recognized and appreciated that image projections may greatly enhance the efficiency of determining the condition of a tissue.

FIG. 2 illustrates an example of a process that may be implemented by an image analysis facility in some embodiments to analyze MR data and determine tissue quality. For ease of description, the process 200 will be described in connection with determining the condition of an ACL, but it should be appreciated the embodiments are not limited to determining the condition of an ACL and that some embodiments may determine the condition of other connective tissues in other joints, as aspects of the technology described herein are not limited in this respect.

Prior to the start of the process 200 of FIG. 2, the MR image analysis facility (e.g., facility 122 of FIG. 1) may receive MR image data corresponding to an imaging of a patient, with the MR data being received by the facility as raw MR data or processed MR data. For example, the MR image of a knee may be a 3D volumetric MR image, a series of 2D MR images corresponding to different imaging planes of the knee, or raw complex imaging data. In yet other embodiments, the MR image of the knee may be a processed MR image. In yet other embodiments, the MR image data may be acquired directly by the MR analysis facility.

In some embodiments, process 200 may use a portion of the MR data. For example, process 200 may use the intensity, phase, spatial frequency, and/or relaxation time associated with the MR data. In other embodiments, process 200 may use all portions of the MR data. In yet other embodiments, process 200 may use different portions of the MR data for determining the condition at different locations of the MR image.

In some embodiments, the MR image received by the MR image analysis facility is a segmented portion of a larger image. For example, MR image analysis facility may receive as an input a 3D MR image of an ACL of a patient that has been segmented from a 3D image of the knee of the patient. The image segmenting in such embodiments may be performed in any suitable manner, such as by a user by selecting regions from a 3D image corresponding to the ACL to specify a mask image to be input to the image analysis facility.

In some embodiments, image segmentation may be automated and may be executed using known methods in the art. As an example, an MR image of a knee may be received by the MR image analysis facility and the image analysis facility may first segment a ligament from the image of the joint before generating a projection of the image. The automated image segmentation may include an object detection, object identification, masking, classifying, and may involve detecting a global threshold and/or local thresholds associated with the regions to be segmented, convolution neural networks, region-based segmentation, edge detection segmentation, segmentation based on clustering, or other image analysis processes as the technology described herein is not limited in this respect.

In some embodiments, process 200 of FIG. 2 may begin at act 202 in which the image analysis facility generates a projection based on an MR image of a joint. For example, in some embodiments, the image analysis facility generates a 2D projection of tissue of interest from a 3D MR image of a knee. The 2D projection has non-overlapping pixels corresponding to the voxels of the connective tissue from the 3D image. In other embodiments, the projection may be a 3D projection where the surface voxels may reflect interior values of the tissue.

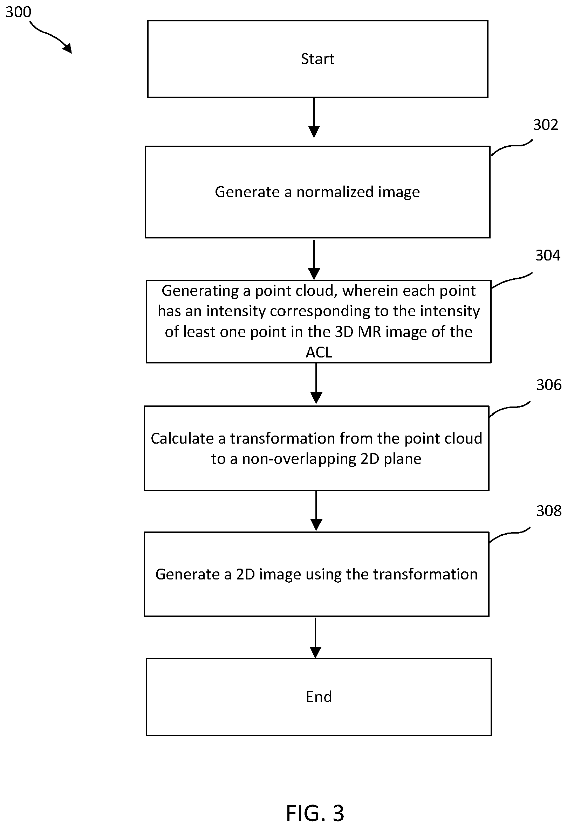

In connection with FIG. 3, the process 300 is described as using the intensity of MR data to generate the 2D projection, however, process 300 may use any portion of the MR data, as described herein. In some embodiments, process 300 may begin at act 302, after receiving a 3D MR image, in which the image analysis facility generates a normalized image from the 3D MR image. For example, the image analysis facility may normalize the 3D MR image with reference to one of the tissues depicted in the MR image. Tissues with minimal variation in signal intensity may be used to generate a reference value for normalizing the 3D MR image data. In some embodiments, the 3D MR image data may be normalized to an intensity associated with a femoral cortical bone of the patient. In other embodiments, the 3D MR image data may be normalized relative to a predetermined value. In yet other embodiments, the user may select a portion of the image to be used to normalize the 3D MR image data.

In some embodiments, when the MR image is acquired as a series of 2D images to create an image stack, the normalization may be calculated for each frame relative to a tissue that is observable in all frames and that has stable image intensity. For example, the ACL tissue in a series of images may be normalized to the intensity of a femoral cortical bone depicted within the same image frame as the respective portion of the ACL tissue.

At act 304, the image analysis facility generates a point cloud to represent the voxels in the 3D MR image, in accordance with some embodiments. In the point cloud, each voxel is represented by a coordinate, corresponding to the spatial position of the signal, and a signal value associated with the MR signal at the coordinate in the 3D MR image. In some embodiments, the signal value may be complex corresponding to the intensity and phase of the MR signal. In other embodiments, the signal value may be a real value corresponding to the intensity or magnitude of the MR signal associated with the coordinates.

The points in the point cloud may include additional calculations based on the 3D MR image, such as, averaging, weighting, filtering, noise correction, thresholding, and other image processing techniques. As such, the resulting point cloud may have fewer points for a corresponding tissue then there are voxels associated with the tissue in the 3D MR image. In other embodiments, the number of points in the point cloud may correspond to the number of voxels associated with the tissue in the 3D MR image. In yet other embodiments, the point cloud may include more points than the number of voxels associated with the tissue in the 3D MR image. For example, an upsampling technique may be used to generate more points in the point cloud than in the received 3D MR image.

At act 306, the image analysis facility calculates a transformation from the point cloud to a non-overlapping 2D plane. For example, the transformation may correspond to a mapping between a 3D space and a 2D space such that each point in the point cloud has a respective location in the 2D projection. In some embodiments, the projection may be generated by first grouping the points into respective sagittal planes associated with the tissue. Next, the sagittal planes may be arranged adjacent to one another in a 2D projection, such that the nearest neighboring points within the sagittal planes of the MR image are nearest neighboring points in the 2D projection.

In other embodiments, the MR signals from the interior of a tissue may be grouped according to the surface coordinate that they are closest to. Then each of the groups may be statistically processed and the data associated with the surface of the tissue set to the result of the statistical processing. For example, the processing may include an average value, standard deviation, maximum, minimum.

At act 308, the image analysis facility generates the 2D projection using the calculated transformation. The resulting projection image represents each of the voxels from the 3D image with a location in the 2D projection. The 2D projection may be represented as an image where symbols are used at each location to represent the points. In some embodiments, the symbols may be circular and characterized by a number of pixels. The image analysis facility may include additional transformations associated with generating the 2D image. For example, the image analysis facility may increase or decrease the distance between adjacent symbols in the 2D image or resize the symbols of the displayed image.

Referring again to FIG. 2, at act 204, the image analysis facility determines values indicative of a condition of the tissue at locations depicted in the projection. In some embodiments, a location is an area or region of the projection that includes multiple points. For example, the number of points associated with each location may be equal, or some regions may be associated with more points than other regions. In some embodiments, the number of points associated with a location in the projection may depend on a desired resolution at that location. For example, some locations of the tissue may correspond to a position of interest in the MR image and may have fewer points associated with a given location around the position, such that the number of locations that represent the position is larger than other positions of the projection. In other embodiments, a location is a single point or pixel.

In some embodiments, the condition may include determining status of tissue health, strength, remodeling, risk of injury, and/or recovery after injury and treatment for a patient. For example, the image analysis facility may use a predetermined threshold value, corresponding to a condition of a patient. Additionally, or alternatively, the image analysis facility may use clinical data associated with the patient to determine a threshold value for a condition of a tissue.

In some embodiments, the image analysis facility may consider the pulse sequence used to acquire the MR data. The threshold value may depend upon the pulse sequence used to acquire the MR data. For example, when a Constructive Interference in the Steady State (CISS) sequence is used, damaged tissue may appear brighter than healthy tissue. CISS is a T2-weighted pulse sequence, as such, the tissue contrast is different in an MR image generated using a T2-weighted pulse sequence relative to an MR image generated using a T1-weighted pulse sequence. As such, the image analysis facility may determine the threshold based, at least in part, on the pulse sequence used to acquire the MR image.

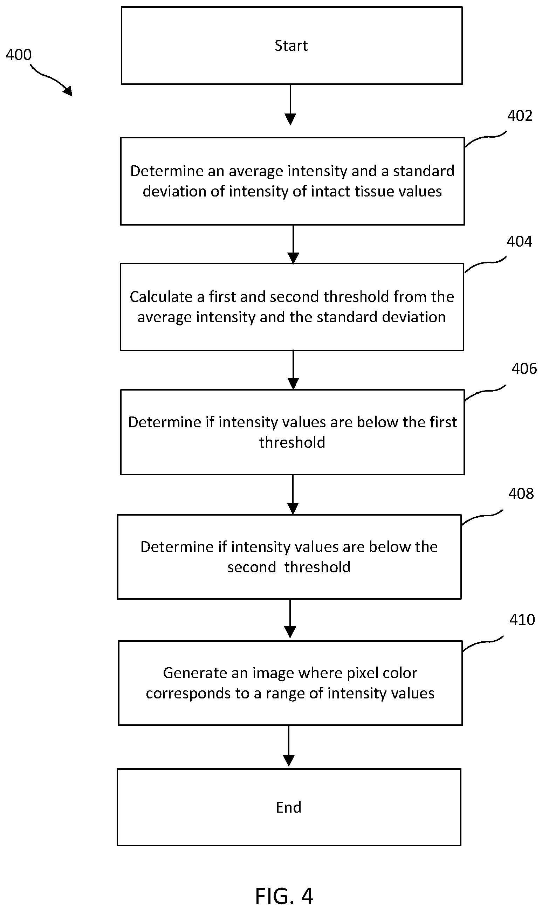

In some embodiments, the process 400 of FIG. 4 may be used to determine values indicative of a condition of a tissue. For ease of description, process 400 will be described in connection with MR data generated using a T2-weighted pulse sequence, but it should be appreciated that embodiments are not limited to determining values indicative of a condition of a tissue from MR data generated using a T2-weighted pulse sequences. The use of signal spatial distribution instead of absolute value enables the current technique to be less magnet and sequence dependent; thus, enables development of algorithms to track tissue healing from a range different sequences and MRI magnets.

In connection with FIG. 4, process 400 is described as determining values indicative of a condition of the tissue at locations depicted in the projection based on the intensity, however, process 400 may determine the values based on any portion of the MR data, as described herein. Process 400 starts at act 402 where an average intensity and a standard deviation of intensity are determined. In some embodiments the average intensity and standard deviation of intensity may be determined from calculations of the average and standard deviation of image points in the projection. For example, the average intensity and standard deviation may be calculated from the normalized values in the 2D projection.

In other embodiments, the average intensity and standard deviation may be determined based on values calculated from a plurality of MR images of healthy tissue. For example, average and standard deviation of intensity for ACL tissue may be determined from reference images acquired on patients that have not experience any ACL injury. The reference MR images may be normalized to the intensity of a tissue, depicted within the joint image, before the reference images are used to generate average and standard deviation values. In embodiments corresponding to determining the average intensity and standard deviation for a healthy ACL ligament (e.g., an intact ACL), the intensity values may be normalized to an intensity associated with a femoral cortical bone or other reference tissue with stable image intensity.

In yet other embodiments, the average and standard deviation of intensity may be determined from a look up table. The look up table may be stored locally to the image analysis facility, on the same local network, or may be an external database accessed through a network such as the network 140 in FIG. 1.

At act 404, a first and second threshold are calculated from the average intensity and the standard deviation. For example, the first threshold may correspond to an intensity one standard deviation smaller than the average intensity and the second threshold may correspond to an intensity one standard deviation larger than the average intensity.

At act 406, the image analysis facility determines if the locations in the projection are below a first threshold. For example, if the intensity value at an image location is less than a first threshold, then the image location may be determined to represent superior tissue quality.

At act 408, the image analysis facility determines if the locations in the projection are below the second threshold. For example, if the intensity value at an image location is less than the second threshold and larger than the first threshold, then the image location may be determined to represent normal tissue quality. The image locations with an intensity greater than the second threshold may be determined to represent inferior tissue quality.

At act 410, the image analysis facility generates an image where pixel color corresponds to a range of intensity values. For example, if the intensity values of a first set of locations in the projection were less than the first threshold, then pixels associated with the first set of locations are assigned a first color; if the intensity values of a second set of locations in the projection were greater than the first threshold but less than the second threshold, then pixels associated with the second set of locations in the projection are assigned as second color; and if the intensity values of a third set of locations in the projection are greater than the second threshold, then pixels associated with the third set of locations are assigned a third color.

In some embodiments, there may be more than two thresholds. For example, the image analysis facility may compare the values to 1, 3, 4, 5, or greater than 10 thresholds. In some embodiments the thresholds may be evenly distributed across a range of intensity values. In other embodiments, the thresholds may be logarithmically distributed across a range of intensity. In yet other embodiments, the thresholds may have a first spacing for a range of intensity values less than the average intensity and a second spacing for a range of intensity values greater than the average intensity. Additionally, or alternatively, the thresholds may be used in non-strict inequalities (e.g. ≥ and ≤) and/ strict inequalities (e.g. > and <) for determining the condition of the tissue.

Referring once again to FIG. 2, at act 206, the image analysis facility outputs the determined condition of the tissue. In some embodiments, the determined condition of the tissue is output as a healing map. For example, a tricolor scheme may be used to indicate the condition at each location in the projection. In some embodiments, the locations in the projection may be yellow, orange, or red to indicate the condition of the tissue represented by that location.

In other embodiments, a two-color scheme or other multi-color scheme may be used to indicate the condition of a tissue. Additionally, or alternatively, the size, opacity, shape of the symbols representing the locations in the projection, and/or other display parameter may be used to indicate the condition of a tissue.

Once the tissue condition information is output, the process 200 of FIG. 2 ends. Following the process, in some cases, the healing map may be used by a clinician to make clinical determinations. For example, a clinician may use the healing map to determine the status of tissue health, the strength of ligament tissue, remodeling of the tissue, risk of injury, and/or recovery after injury and treatment for a patient. Additionally, or alternatively, the healing map may be used to aid in other clinical determinations as aspects of the technology described herein are not limited in this respect. The healing map may be stored in one or more data stores, such as in an electronic health record, a picture archiving and communication system (PACS), or other data store. The healing map may also be output for display to a clinician in some cases.

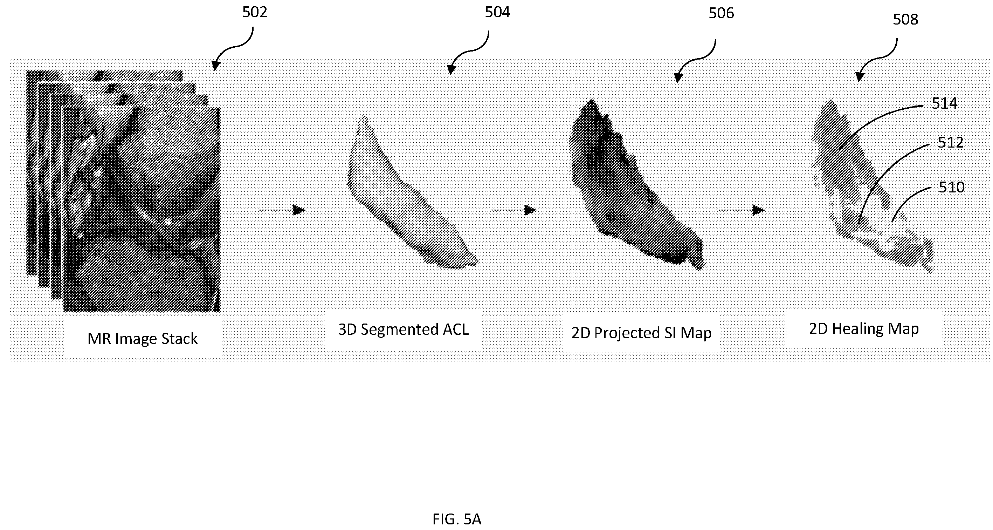

FIG. 5A is an illustrative example of a process of generating a healing map from a MR image, in accordance with some embodiments described herein. The MR image stack 502 was acquired using a CISS sequence to image the knee of a patient following an ACL surgery. MR image stack depicts the ACL and surrounding tissue of the knee. The ACL portions of the image stack were manually segmented from the sagittal CISS image stacks to generate 3D segmented ACL 504. A point cloud (not pictured) may then be generated from the 3D segmented ACL, in accordance with some aspects of the technology described herein.

The 2D projected signal intensity (SI) map 506, may be generated in accordance with projection techniques such as those described herein, e.g., the process of FIG. 3. In the illustrated embodiment of FIG. 5A, the SI map is generated from a point cloud (not pictured) where the intensity values are normalized to the patient specific grayscale value of the femoral cortical bone, depicted in MR image stack 502. After the normalization, all the sagittal slices of the ligament represented in the point cloud are projected into a single 2D image 506.

Healing map 508 is generated by comparing the normalized signal intensity (I_{2D}) of the locations in the 2D projected SI map 506 to reference values determined from the average (I_{SD}) and standard deviation (SD) of intensity from MR images of intact ACLs. For locations with a normalized signal intensity at least one standard deviation greater than the average intensity, for intact ACLs, the locations are determined to be inferior tissue quality and are color coded light gray, 510; for locations with a normalized signal intensity within one standard deviation of the average intensity, for intact ACLs, the locations are determined to be normal tissue quality and are color coded gray, 512; and for locations with a normalized signal intensity at least one standard deviation smaller than the average intensity, for intact ACLs, the locations are determined to be superior tissue quality and are color coded dark gray, 514. The determinations are illustrated by the equations below, in accordance with some embodiments. In other embodiments, the condition of the tissue may be determined using other techniques, as described herein.
*inferior tissue quality*: ${I}_{2 D} > {I}_{avg} + SD$
*normal tissue qualtiy*: ${I}_{avg} - SD < {I}_{2 D} < {I}_{avg} + SD$
*superior tissue quality*: ${I}_{2 D} < {I}_{avg} - SD$

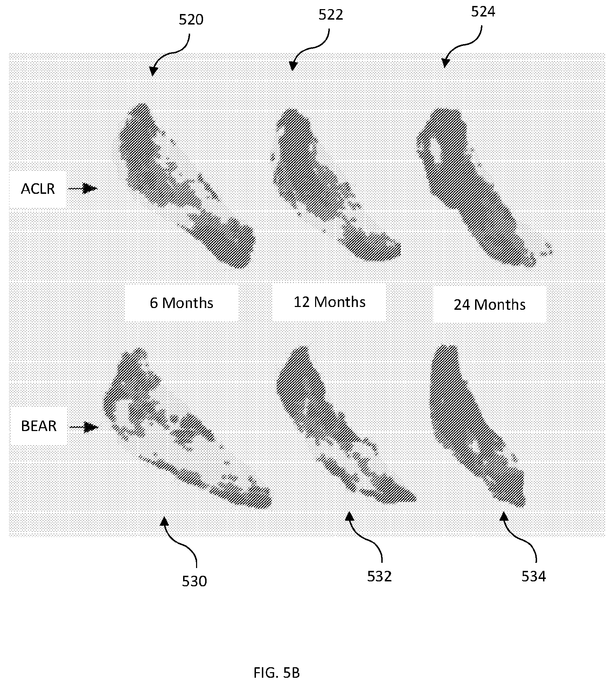

FIG. 5B is an illustrative example of postoperative healing maps illustrating tissue remodeling over time. On the top, healing maps 520, 522, and 524 correspond to 6, 12, and 24 months after an ACL reconstruction (ACLR). On the bottom, healing maps 530, 532, and 534 correspond to 6, 12, and 24 months after a bridge-enhanced ACL repair (BEAR). For both the ACLR and BEAR more than half of the treated ligament consisted of low-quality (high signal) tissue, mainly across the mid-substance of the ligament (520 and 530). The low-quality tissue was gradually remodeled into higher quality tissue with less than a quarter of the ligament volume having normalized signal intensities higher than the intact average ACL at 24 months after surgery (524 and 534).

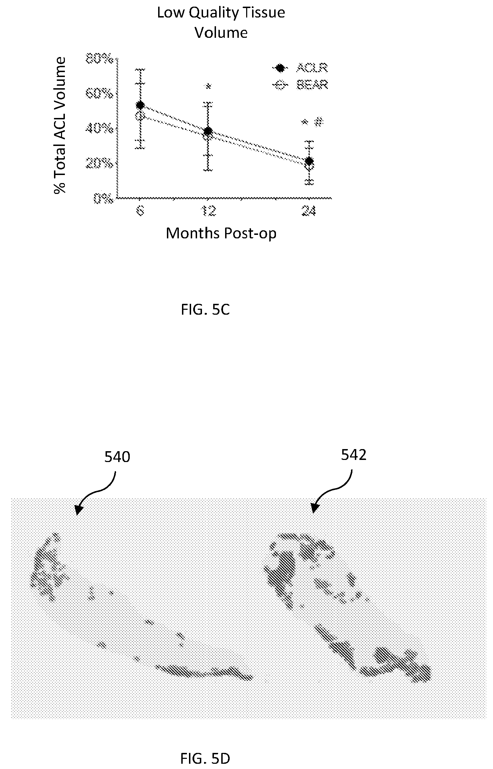

Both ACLR and BEAR healing maps, as illustrated in FIG. 5B, illustrate a clear progression of tissue healing from 6 to 24 month. This postoperative changes in the volume of the low-quality tissue as a function of months after surgery, for both the ACLR and BEAR healing maps, is plotted in FIG. 5C.

FIG. 5D illustrates two healing maps, generated from MR images acquired six months after ACL surgery, in patients that experienced subsequent ACL reinjury and required revision surgery. Comparing the healing maps in FIG. 5D with those in FIG. 5B, the healing maps 540 and 542 have large regions of low-quality tissue spread across the treated ligament.

Techniques operating according to the principles described herein may be implemented in any suitable manner. Included in the discussion above are a series of flowcharts showing the steps or acts of various processes that analyze MR data of a joint to determine the condition of a tissue that may be used by a clinician or researcher to determine tissue health, strength, remodeling, risk of injury, and/or recovery after injury and treatment for a patient. The processing and decision blocks of the flowcharts above represent steps and acts that may be included in algorithms that carry out these various processes. Algorithms derived from these processes may be implemented as software integrated with and directing the operation of one or more single- or multi-purpose processors, may be implemented as functionally-equivalent circuits such as a Digital Signal Processing (DSP) circuit or an Application-Specific Integrated Circuit (ASIC), or may be implemented in any other suitable manner. It should be appreciated that the flowcharts included herein do not depict the syntax or operation of any particular circuit or of any particular programming language or type of programming language. Rather, the flowcharts illustrate the functional information one skilled in the art may use to fabricate circuits or to implement computer software algorithms to perform the processing of a particular apparatus carrying out the types of techniques described herein. It should also be appreciated that, unless otherwise indicated herein, the particular sequence of steps and/or acts described in each flowchart is merely illustrative of the algorithms that may be implemented and can be varied in implementations and embodiments of the principles described herein.

Accordingly, in some embodiments, the techniques described herein may be embodied in computer-executable instructions implemented as software, including as application software, system software, firmware, middleware, embedded code, or any other suitable type of computer code. Such computer-executable instructions may be written using any of a number of suitable programming languages and/or programming or scripting tools, and also may be compiled as executable machine language code or intermediate code that is executed on a framework or virtual machine.

When techniques described herein are embodied as computer-executable instructions, these computer-executable instructions may be implemented in any suitable manner, including as a number of functional facilities, each providing one or more operations to complete execution of algorithms operating according to these techniques. A "functional facility," however instantiated, is a structural component of a computer system that, when integrated with and executed by one or more computers, causes the one or more computers to perform a specific operational role. A functional facility may be a portion of or an entire software element. For example, a functional facility may be implemented as a function of a process, or as a discrete process, or as any other suitable unit of processing. If techniques described herein are implemented as multiple functional facilities, each functional facility may be implemented in its own way; all need not be implemented the same way. Additionally, these functional facilities may be executed in parallel and/or serially, as appropriate, and may pass information between one another using a shared memory on the computer(s) on which they are executing, using a message passing protocol, or in any other suitable way.

Generally, functional facilities include routines, programs, objects, components, data structures, etc. that perform particular tasks or implement particular abstract data types. Typically, the functionality of the functional facilities may be combined or distributed as desired in the systems in which they operate. In some implementations, one or more functional facilities carrying out techniques described herein may together form a complete software package. These functional facilities may, in alternative embodiments, be adapted to interact with other, unrelated functional facilities and/or processes, to implement a software program application, for example as a software program application such as an image analysis facility.

Some exemplary functional facilities have been described herein for carrying out one or more tasks. It should be appreciated, though, that the functional facilities and division of tasks described is merely illustrative of the type of functional facilities that may implement the exemplary techniques described herein, and that embodiments are not limited to being implemented in any specific number, division, or type of functional facilities. In some implementations, all functionality may be implemented in a single functional facility. It should be appreciated that, in some implementations, some of the functional facilities described herein may be implemented together with or separately from others (i.e., as a single unit or separate units), or some of these functional facilities may not be implemented.

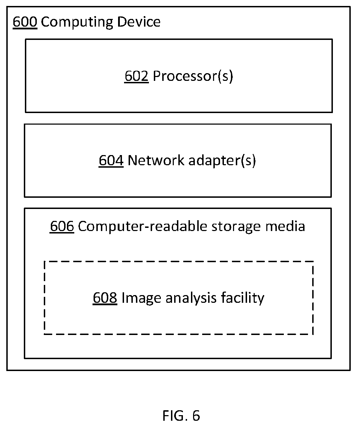

Computer-executable instructions implementing the techniques described herein (when implemented as one or more functional facilities or in any other manner) may, in some embodiments, be encoded on one or more computer-readable media to provide functionality to the media. Computer-readable media include magnetic media such as a hard disk drive, optical media such as Compact Disk (CD) or a Digital Versatile Disk (DVD), a persistent or non-persistent solid-state memory (e.g., Flash memory, Magnetic RAM, etc.), or any other suitable storage media. Such a computer-readable medium may be implemented in any suitable manner, including as computer-readable storage media 606 of FIG. 6 described below (e.g., as a portion of a computing device 600) or as a stand-alone, separate storage medium. As used herein, "computer-readable media" (also called "computer-readable storage media") refers to tangible storage media. Tangible storage media are non-transitory and have at least one physical, structural component. In a "computer-readable medium," as used herein, at least one physical, structural component has at least one physical property that may be altered in some way during a process of creating the medium with embedded information, a process of recording information thereon, or any other process of encoding the medium with information. For example, a magnetization state of a portion of a physical structure of a computer-readable medium may be altered during a recording process.

In some, but not all, implementations in which the techniques may be embodied as computer-executable instructions, these instructions may be executed on one or more suitable computing device(s) operating in any suitable computer system, including the exemplary computer system of FIG. 1, or one or more computing devices (or one or more processors or one or more computing devices) may be programmed to execute the computer-executable instructions. A computing device or processor may be programmed to execute instructions when the instructions are stored in a manner accessible to the computing device or processor, such as in a data store (e.g., an on-chip cache or instruction register, a computer-readable storage medium accessible via a bus, a computer-readable storage medium accessible via one or more networks and accessible by the device/processor, etc.). Functional facilities comprising these computer-executable instructions may be integrated with and direct the operation of a single multi-purpose programmable digital computing device, a coordinated system of two or more multi-purpose computing device sharing processing power and jointly carrying out the techniques described herein, a single computing device or coordinated system of computing devices (co-located or geographically distributed) dedicated to executing the techniques described herein, one or more Field-Programmable Gate Arrays (FPGAs) for carrying out the techniques described herein, or any other suitable system.

FIG. 6 illustrates one exemplary implementation of a computing device in the form of a computing device 600 that may be used in a system implementing techniques described herein, although others are possible. It should be appreciated that FIG. 6 is intended neither to be a description of necessary components for a computing device to operate as an image analysis facility in accordance with the principles described herein, nor a comprehensive depiction.

Computing device 600 may comprise at least one processor 602, a network adapter 604, and computer-readable storage media 606. Computing device 600 may be, for example, a desktop or laptop personal computer, a personal digital assistant (PDA), a smart mobile phone, or any other suitable computing device. Network adapter 604 may be any suitable hardware and/or software to enable the computing device 600 to communicate wired and/or wirelessly with any other suitable computing device over any suitable computing network. The computing network may include wireless access points, switches, routers, gateways, and/or other networking equipment as well as any suitable wired and/or wireless communication medium or media for exchanging data between two or more computers, including the Internet. Computer-readable media 606 may be adapted to store data to be processed and/or instructions to be executed by processor 602. Processor 602 enables processing of data and execution of instructions. The data instructions may be stored on the computer-readable storage media 606.

The data and instructions stored on computer-readable storage media 606 may comprise computer-executable instructions implementing techniques which operate according to the principles described herein. In the example of FIG. 6, computer-readable storage media 606 stores computer-executable instructions implementing various facilities and storing various information as described above. Computer-readable storage media 606 may store image analysis facility 608 configured to derive information indicative of a condition of a patient from MR data.

While not illustrated in FIG. 6, a computing device may additionally have one or more components and peripherals, including input and output devices. These devices can be used, among other things, to present a user interface. Examples, of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computing device may receive input information through speech recognition or in other audible format.

Embodiments have been described where the techniques are implemented in circuitry and/or computer-executable instructions. It should be appreciated that some embodiments may be in the form of a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Various aspects of the embodiments described above may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects describe in other embodiments.

Also, the phraseology and terminology used herein is for the purpose of description and should not be regarded as limiting. The use of "including," "comprising," "having," "containing," "involving," and variations thereof herein, is meant to encompass the items listed thereafter and equivalents thereof as well as additional items.

The word "exemplary" is used herein to mean serving as an example, instance, or illustration. Any embodiment, implementation, process, feature, etc. described herein as exemplary should therefore be understood to be an illustrative example and should not be understood to be a preferred or advantageous example unless otherwise indicated.

Having thus described several aspects of at least one embodiment, it is to be appreciated that various alternations, modifications, and improvements will readily occur to those skilled in the art. Such alterations, modifications, and improvements are intended to be part of this disclosure and are intended to be within the scope of the principles described herein. Accordingly, the foregoing description and drawings are by way of example only, the invention being defined by the appended claims.

## Claims

1. A computer-implemented method of determining a condition of a connective tissue of a patient from analysis of a magnetic resonance image, the method comprising:
generating (202) a projection from a 3D magnetic resonance image depicting the tissue, wherein
generating the projection comprises:
calculating a transformation to project points from the magnetic resonance image into non-overlapping coordinates in a 2D plane; and
generating the projection as a 2D image using the transformation to determine a value at each point in the projection based on at least one value at a position of the magnetic resonance image corresponding to the point in the projection;
the method further comprising:
determining (204), for each of a plurality of locations in the projection, the condition of the tissue at a location, wherein determining the condition of the tissue at the location comprises determining the condition based at least in part on at least one value of the projection at the location; and
outputting (206) the determined condition at the plurality of locations of the projection.

2. The method of claim 1, wherein the magnetic resonance image depicting the tissue comprises a magnetic resonance image depicting a knee of the patient,
optionally wherein the magnetic resonance image depicting the knee comprises a magnetic resonance image depicting a connective tissue of the knee of the patient.

3. The method of claim 2, wherein the magnetic resonance image depicting the connective tissue comprises a magnetic resonance image depicting an Anterior Cruciate Ligament (ACL) of the patient.

4. The method of claim 3, wherein the magnetic resonance image depicts only the ACL of the patient.

5. The method of claim 4, further comprising:
segmenting a first magnetic resonance image of the knee of the patient to yield the magnetic resonance image depicting only the ACL of the patient.

6. The method of claim 1, wherein generating the projection from the magnetic resonance image comprises:
grouping a plurality of points from the magnetic resonance image into a plurality of sagittal planes and arranging the plurality of sagittal planes adjacent to one another.

7. The method of claim 1, further comprising normalizing signal intensity of the magnetic resonance image, wherein normalizing the signal intensity comprises normalizing the signal intensity to an intensity in the image associated with a reference tissue.

8. The method of claim 7, wherein the reference tissue comprises an intensity associated with a femoral cortical bone of the patient.

9. The method of claim 1, wherein calculating the transformation comprises calculating the transformation such that nearest neighboring points within slices of the magnetic resonance image are nearest neighboring points in the 2D image.

10. The method of claim 1, wherein:
the method further comprises:
determining an average image intensity of intact tissue values;
calculating at least one threshold indicative of the condition of the tissue using the average image intensity; and
determining the condition of the tissue at a location of the plurality of locations in the projection comprises:
comparing one or more values of the projection at the location to the at least one threshold; and
determining the condition of the tissue based on a result of the comparing.

11. The method of claim 10, wherein the at least one threshold comprises a first threshold intensity that is a standard deviation larger than the average intensity,
optionally wherein the at least one threshold comprises a second threshold intensity that is a standard deviation less than the average intensity.

12. The method of claim 10, wherein determining the condition of the tissue at a location comprises categorizing tissue quality at the location, based on one or more values of the projection at the location, as being of above-average, average, or below-average tissue quality.

13. The method of claim 10, wherein outputting the determined condition at the plurality of locations comprises:
visually annotating the projection at each location based on the categorized tissue quality at the location; and
outputting the annotated projection.

14. The method of claim 13, wherein annotating the projection comprises assigning a color to each location indicative of the categorized tissue quality at the location.

15. A computer system, comprising:
at least one processor; and
a non-transitory computer-readable storage medium storing executable instructions that,
when executed by the at least one processor, cause the at least one processor to perform a method of determining a condition of a connective tissue of a patient from analysis of a magnetic resonance image, the method comprising:
generating (202) a projection from a 3D magnetic resonance image depicting the tissue, wherein generating the projection comprises:
calculating a transformation to project points from the magnetic resonance image into non-overlapping coordinates in a 2D plane; and
generating the projection as a 2D image using the transformation to determine a value at each point in the projection based on at least one value at a position of the magnetic resonance image corresponding to the point in the projection;
the method further comprising:
determining(204), for each of a plurality of locations in the projection, the condition of the tissue at a location, wherein determining the condition of the tissue at the location comprises determining the condition based at least in part on at least one value of the projection at the location, and
outputting (206) the determined condition at the plurality of locations of the projection.

## Patentansprüche

1. Computerimplementiertes Verfahren zum Bestimmen eines Zustands eines Bindegewebes eines Patienten aus einer Analyse eines Magnetresonanzbildes, wobei das Verfahren umfasst:
Erzeugen (202) einer Projektion aus einem 3D-Magnetresonanzbild, das das Gewebe abbildet, wobei das Erzeugen der Projektion
umfasst:
Berechnen einer Transformation zum Projizieren von Punkten aus dem Magnetresonanzbild in einander nicht überlappende Koordinaten in einer 2D-Ebene; und
Erzeugen der Projektion als 2D-Bild unter Verwendung der Transformation, um einen Wert an jedem Punkt in der Projektion auf der Grundlage mindestens eines Werts an einer Position des Magnetresonanzbildes, die dem Punkt in der Projektion entspricht, zu bestimmen;
wobei das Verfahren ferner umfasst:
Bestimmen (204), für jede einer Mehrzahl von Stellen in der Projektion, des Zustands des Gewebes an einer Stelle, wobei das Bestimmen des Zustands des Gewebes an der Stelle umfasst, den Zustand zumindest teilweise auf der Grundlage mindestens eines Werts der Projektion an der Stelle zu bestimmen; und Ausgeben (206) des bestimmten Zustands an der Mehrzahl von Stellen der Projektion.

2. Verfahren nach Anspruch 1, wobei das Magnetresonanzbild, das das Gewebe abbildet, ein Magnetresonanzbild umfasst, das ein Knie des Patienten abbildet, wobei optional das Magnetresonanzbild, das das Knie abbildet, ein Magnetresonanzbild umfasst, das ein Bindegewebe des Knies des Patienten abbildet.

3. Verfahren nach Anspruch 2, wobei das Magnetresonanzbild, das das Bindegewebe abbildet, ein Magnetresonanzbild umfasst, das ein vorderes Kreuzband (ACL) des Patienten abbildet.

4. Verfahren nach Anspruch 3, wobei das Magnetresonanzbild nur das ACL des Patienten abbildet.

5. Verfahren nach Anspruch 4, ferner umfassend: Segmentieren eines ersten Magnetresonanzbildes des Knies des Patienten, um das Magnetresonanzbild zu erhalten, das nur das ACL des Patienten abbildet.

6. Verfahren nach Anspruch 1, wobei das Erzeugen der Projektion aus dem Magnetresonanzbild umfasst:
Gruppieren einer Mehrzahl von Punkten aus dem Magnetresonanzbild in eine Mehrzahl von Sagittalebenen und Anordnen der Mehrzahl von Sagittalebenen einander benachbart.

7. Verfahren nach Anspruch 1, ferner umfassend ein Normalisieren einer Signalintensität des Magnetresonanzbildes, wobei das Normalisieren der Signalintensität umfasst, die Signalintensität auf eine Intensität in dem Bild zu normalisieren, die einem Referenzgewebe zugeordnet ist.

8. Verfahren nach Anspruch 7, wobei das Referenzgewebe eine Intensität umfasst, die einem kortikalen Femurknochen des Patienten zugeordnet ist.

9. Verfahren nach Anspruch 1, wobei das Berechnen der Transformation umfasst, die Transformation derart zu berechnen, dass nächstbenachbarte Punkte innerhalb von Schichten des Magnetresonanzbildes nächstbenachbarte Punkte in dem 2D-Bild sind.

10. Verfahren nach Anspruch 1, wobei:
das Verfahren ferner umfasst:
Bestimmen einer durchschnittlichen Bildintensität von Werten intakten Gewebes;
Berechnen mindestens eines Schwellenwerts, der den Zustand des Gewebes angibt, unter Verwendung der durchschnittlichen Bildintensität; und
das Bestimmen des Zustands des Gewebes an einer Stelle der Mehrzahl von Stellen in der Projektion umfasst:
Vergleichen eines oder mehrerer Werte der Projektion an der Stelle mit dem mindestens einen Schwellenwert; und
Bestimmen des Zustands des Gewebes auf der Grundlage eines Ergebnisses des Vergleichens.

11. Verfahren nach Anspruch 10, wobei der mindestens eine Schwellenwert eine erste Schwellenintensität umfasst, die um eine Standardabweichung größer als die durchschnittliche Intensität ist, wobei optional der mindestens eine Schwellenwert eine zweite Schwellenintensität umfasst, die um eine Standardabweichung kleiner als die durchschnittliche Intensität ist.

12. Verfahren nach Anspruch 10, wobei das Bestimmen des Zustands des Gewebes an einer Stelle umfasst, die Gewebequalität an der Stelle auf der Grundlage eines oder mehrerer Werte der Projektion an der Stelle als überdurchschnittliche, durchschnittliche oder unterdurchschnittliche Gewebequalität zu kategorisieren.

13. Verfahren nach Anspruch 10, wobei das Ausgeben des bestimmten Zustands an der Mehrzahl von Stellen umfasst:
visuelles Annotieren der Projektion an jeder Stelle auf der Grundlage der kategorisierten Gewebequalität an der Stelle; und
Ausgeben der annotierten Projektion.

14. Verfahren nach Anspruch 13, wobei das Annotieren der Projektion umfasst, jeder Stelle eine Farbe zuzuordnen, die die kategorisierte Gewebequalität an der Stelle angibt.

15. Computersystem, umfassend:
mindestens einen Prozessor; und
ein nichtflüchtiges computerlesbares Speichermedium, das ausführbare Anweisungen speichert, die bei Ausführung durch den mindestens einen Prozessor den mindestens einen Prozessor veranlassen, ein Verfahren zum Bestimmen eines Zustands eines Bindegewebes eines Patienten aus einer Analyse eines Magnetresonanzbildes durchzuführen, wobei das Verfahren umfasst:
Erzeugen (202) einer Projektion aus einem 3D-Magnetresonanzbild, das das Gewebe abbildet, wobei das Erzeugen der Projektion umfasst:
Berechnen einer Transformation zum Projizieren von Punkten aus dem Magnetresonanzbild in einander nicht überlappende Koordinaten in einer 2D-Ebene; und
Erzeugen der Projektion als 2D-Bild unter Verwendung der Transformation, um einen Wert an jedem Punkt in der Projektion auf der Grundlage mindestens eines Werts an einer Position des Magnetresonanzbildes, die dem Punkt in der Projektion entspricht, zu bestimmen;
wobei das Verfahren ferner umfasst:
Bestimmen (204), für jede einer Mehrzahl von Stellen in der Projektion, des Zustands des Gewebes an einer Stelle, wobei das Bestimmen des Zustands des Gewebes an der Stelle umfasst, den Zustand zumindest teilweise auf der Grundlage mindestens eines Werts der Projektion an der Stelle zu bestimmen, und Ausgeben (206) des bestimmten Zustands an der Mehrzahl von Stellen der Projektion.

## Revendications

1. Procédé mis en œuvre par ordinateur pour déterminer un état d'un tissu conjonctif d'un patient à partir de l'analyse d'une image par résonance magnétique, le procédé comprenant :
la génération (202) d'une projection à partir d'une image par résonance magnétique 3D représentant le tissu, dans lequel la génération de la projection
comprend :
le calcul d'une transformation pour projeter des points de l'image par résonance magnétique en des coordonnées non chevauchantes dans un plan 2D ; et
la génération de la projection sous la forme d'une image 2D en utilisant la transformation pour déterminer une valeur en chaque point de la projection sur la base d'au moins une valeur à une position de l'image par résonance magnétique correspondant au point de la projection ;
le procédé comprenant en outre :
la détermination (204), pour chacun d'une pluralité d'emplacements dans la projection, de l'état du tissu à un emplacement, dans lequel la détermination de l'état du tissu à l'emplacement comprend la détermination de l'état sur la base, au moins en partie, d'au moins une valeur de la projection à l'emplacement ; et la délivrance en sortie (206) de l'état déterminé au niveau de la pluralité d'emplacements de la projection.

2. Procédé selon la revendication 1, dans lequel l'image par résonance magnétique représentant le tissu comprend une image par résonance magnétique représentant un genou du patient, éventuellement dans lequel l'image par résonance magnétique représentant le genou comprend une image par résonance magnétique représentant un tissu conjonctif du genou du patient.

3. Procédé selon la revendication 2, dans lequel l'image par résonance magnétique représentant le tissu conjonctif comprend une image par résonance magnétique représentant un ligament croisé antérieur (LCA) du patient.

4. Procédé selon la revendication 3, dans lequel l'image par résonance magnétique ne représente que le LCA du patient.

5. Procédé selon la revendication 4, comprenant en outre : la segmentation d'une première image par résonance magnétique du genou du patient pour obtenir l'image par résonance magnétique ne représentant que le LCA du patient.

6. Procédé selon la revendication 1, dans lequel la génération de la projection à partir de l'image par résonance magnétique comprend :
le regroupement d'une pluralité de points de l'image par résonance magnétique en une pluralité de plans sagittaux et la disposition de la pluralité de plans sagittaux de manière à ce qu'ils soient adjacents les uns aux autres.

7. Procédé selon la revendication 1, comprenant en outre la normalisation de l'intensité de signal de l'image par résonance magnétique, dans lequel la normalisation de l'intensité de signal comprend la normalisation de l'intensité de signal par rapport à une intensité, dans l'image, associée à un tissu de référence.

8. Procédé selon la revendication 7, dans lequel le tissu de référence comprend une intensité associée à un os cortical fémoral du patient.

9. Procédé selon la revendication 1, dans lequel le calcul de la transformation comprend le calcul de la transformation de telle sorte que des points voisins les plus proches à l'intérieur de coupes de l'image par résonance magnétique soient des points voisins les plus proches dans l'image 2D.

10. Procédé selon la revendication 1, dans lequel :
le procédé comprend en outre :
la détermination d'une intensité d'image moyenne de valeurs de tissu intact ;
le calcul d'au moins un seuil indicatif de l'état du tissu en utilisant l'intensité d'image moyenne ; et
la détermination de l'état du tissu à un emplacement de la pluralité d'emplacements dans la projection comprend :
la comparaison d'une ou plusieurs valeurs de la projection au niveau de l'emplacement avec l'au moins un seuil ; et
la détermination de l'état du tissu sur la base d'un résultat de la comparaison.

11. Procédé selon la revendication 10, dans lequel l'au moins un seuil comprend une première intensité seuil qui est supérieure d'un écart-type à l'intensité moyenne, éventuellement dans lequel l'au moins un seuil comprend une seconde intensité seuil qui est inférieure d'un écart-type à l'intensité moyenne.

12. Procédé selon la revendication 10, dans lequel la détermination de l'état du tissu à un emplacement comprend la catégorisation d'une qualité de tissu à l'emplacement, sur la base d'une ou plusieurs valeurs de la projection à l'emplacement, comme étant d'une qualité de tissu supérieure à la moyenne, moyenne ou inférieure à la moyenne.

13. Procédé selon la revendication 10, dans lequel la délivrance en sortie de l'état déterminé au niveau de la pluralité d'emplacements comprend :
l'annotation visuelle de la projection à chaque emplacement sur la base de la qualité de tissu catégorisée à l'emplacement ; et
la délivrance en sortie de la projection annotée.

14. Procédé selon la revendication 13, dans lequel l'annotation de la projection comprend l'attribution, à chaque emplacement, d'une couleur indicative de la qualité de tissu catégorisée à l'emplacement.

15. Système informatique, comprenant :
au moins un processeur ; et
un support de stockage non transitoire lisible par ordinateur sur lequel sont stockées des instructions exécutables qui, lorsqu'elles sont exécutées par l'au moins un processeur, amènent l'au moins un processeur à exécuter un procédé pour déterminer un état d'un tissu conjonctif d'un patient à partir de l'analyse d'une image par résonance magnétique, le procédé comprenant :
la génération (202) d'une projection à partir d'une image par résonance magnétique 3D représentant le tissu, dans lequel la génération de la projection comprend :
le calcul d'une transformation pour projeter des points de l'image par résonance magnétique en des coordonnées non chevauchantes dans un plan 2D ; et
la génération de la projection sous la forme d'une image 2D en utilisant la transformation pour déterminer une valeur en chaque point de la projection sur la base d'au moins une valeur à une position de l'image par résonance magnétique correspondant au point de la projection ;
le procédé comprenant en outre :
la détermination (204), pour chacun d'une pluralité d'emplacements dans la projection, de l'état du tissu à un emplacement, dans lequel la détermination de l'état du tissu à l'emplacement comprend la détermination de l'état sur la base, au moins en partie, d'au moins une valeur de la projection à l'emplacement, et la délivrance en sortie (206) de l'état déterminé au niveau de la pluralité d'emplacements de la projection.
